# EUROPEAN PATENT SPECIFICATION

(11) **EP 0 659 068 B1**
(45) Date of publication and mention of the grant of the patent: **04.12.1996**
(21) Application number: 93922665.0
(22) Date of filing: 10.09.1993
(51) Int. Cl.: A61K 7/16, A61K 7/18

(54) **MOUTH-CARE PRODUCTS WITH ANTI-BACTERIAL ACTIVITY**
MUNDPFLEGEMITTEL ANTIBAKTERIELLER AKTIVITÄT
PRODUITS D'HYGIENE BUCCALE AYANT UNE ACTIVITE ANTI-BACTERIENNE

(30) Priority: 10.09.1992 EP 92202773
(43) Date of publication of application: 28.06.1995
(73) Proprietor: Sara Lee/DE N.V., 3532 AA Utrecht (NL)
(72) Inventor: TIMMER, Christiena, Jannie, NL-3811 WK Amersfoort (NL)
(74) Representative: Smulders, Theodorus A.H.J., Ir.
(86) International application number: NL9300185
(87) International publication number: WO9405251

(56) References cited:
- EP-A- 0 140 498
- EP-A- 0 181 578
- EP-A- 0 342 486
- WO-A-89/12399
- DATABASE WPI Derwent Publications Ltd., London, GB; AN 92-300523 & CA,A,2 055 984 (HAARMANN & REIMER CORP) 22 June 1992

## Description

The present invention is related to mouth-care products with anti-bacterial activity. More specifically, the present invention is related to mouth-care products containing an effective amount of at least one lantibiotic and at least one compound yielding fluoride ions.

Bacteria that are present in the oral cavity lead to dental plaque formation. Plaque is considered to be the prime etiological factor in the development of caries, and it is also implicated in periodontal diseases. Plaque, when not sufficiently removed, develops into calculus, a hard, mineralised formation which deposits on the enamel surface of the teeth. There is also an association between the presence of calculus and the incidence of periodontal diseases. Serious teeth problems, like caries, gingivitis and other periodontal diseases, can be the result of formation of plaque and calculus.

In the prior art many different lines of approach have been used to find solutions to the problems in the field of oral care.

The first attempts were to remove plaque mechanically, using toothbrush and a dentrifice with polishing compounds. Abrasive and polishing compounds have many times been proposed to help the removal of plaque.

US patents 3 927 201 and 3 927 202 describe dentifrice preparations comprising alkali metal phosphate salts as polishing agents. EP 0 097 476 describes β-phase calcium pyrophosphates as dentifrice abrasive. According to US patent 4 340 583 also silicon dioxide compounds are suitable abrasive agents. US patent 3 070 510 discloses dentifrice compositions comprising a resinous compound acting as an abrasive and US 3 935 304 describes sodium bicarbonate.

Regular brushing aids in preventing a rapid build-up of plaque, but even regular brushing is not sufficient to remove all of the plaque which adhere to the teeth.

Antiplaque dentifrice's, based on special chemical compounds, has been described next. Examples of these special ingredients are quaternary ammonium compounds, described in US 4 323 551, sanquinarine (GB 2 042 336), Triclosan (5-chloro-2-(2,4-dichlorophenoxy)phenol) described in EP 0 161 899, p-aminobenzoic acid (US 3 427 380) and many others.

As plaque is developed by bacteria in the oral cavity, is has been proposed to use bactericides as antiplaque agents. The most important ingredient is chlorhexidine, described in many patents, for example DE 1 084 876. A disadvantage of this compound is the discoloration of the teeth. Compounds like bis-biguanides (US 4 067 962) and alexidine (EP 0 004 719) did not stain the teeth, but proved to be insufficiently effective too.

However, a reduction of the bacterial activity in the oral cavity is most important, as this activity is a major cause of most problems related to oral hygienic conditions.

Surprisingly it has now been found that plaque can be effectively be removed by addition of specific bactericine compounds in combination with at least one compound yielding fluoride ions, more in particular an alkali metal fluoride, to mouth-care products used to take care of the teeth and oral health.

This special group of bactericine compounds, suitable to diminish the bacterial activity in the oral cavity corresponds with the group of lantibiotics.

The term lantibiotics has been described in an article in Nature, vol.333, p.276 (1988). It defines a group of polycyclic polypeptides or small proteins, produced by micro-organisms, with antibiotic, i.e., antibacterial and antimicrobial activity. The term 'small' in this case refers to a number of amino acids not exceeding fifty.

Lantibiotics contain intrachain sulfide bridges, called lanthionine units, formed from the thioether groups of the amino acids lanthionine and β-methyllanthionine.

Lantibiotics have been known since several years. Nisin, one of the first lantibiotic to be discovered, is known to act as an antimalarian drug, it releases lysosomal enzymes and lyses erythrocytes (J.Am.Chem.Soc., 93:18, p. 4635 (1971)). Nisin is in present days used as a food preservative (Applied and Environmental Microbiology, vol.56, no.8, pp.2551-2558 (1990)).

European patent application 0 342 486 describes the antibiotic compound gallidermin, also one of the lantibiotics. In this patent application it is described that gallidermin is active against Propionibacterium acnes and it can be used, preferably topically, in pharmaceutical preparations to treat bacterial infections, in particular in skin infections such as eczema, cellulitis and acne. The anti-acne activity of gallidermin is also described in several other publications. Eur.J.Biochem., 177, 53-59 (1988) discloses the activity of gallidermin against Propionibacteria. FEMS Microbiology Letters 58, 263-268 (1989) describes the suitability of both gallidermin and epidermin in the treatment of skin disorders associated with acne. The already cited article in Nature, vol.333, pp.276-278 (1988) describes the activity of epidermin against Gram-positive bacteria. Epidermin and gallidermin are therefore known to be of therapeutic value in topical treatment of acne.

WO-A 91/07164 discloses the use of lantibiotics in cosmetic deodorants. The antibacterial activity of the lantibiotics is used therein to reduce the bacterial flora on the skin, thereby reducing the conversion of odourless perspiration compounds into unpleasantly smelling compounds. All the known applications of the lantibiotics are therefore topical, directed to the handling of skin related problems.

In WO-A 89/12399 bacteriocine compositions have been described comprising lanthionine containing compound and non-bactericidal agents, such as a chelating agent. The use thereof in mouth care products has also been disclosed.

It was surprisingly found that the addition of one or more lantibiotics in combination with at least one compound yielding fluoride ions, to mouth-care products leads to an effective inactivation of the bacteria in the oral cavity and hence reduces the deposition of dental plaque. The use of lantibiotics in combination with fluoride not only reduces teeth problems, like the occurrence of calculus and caries, but especially reduces the occurrence of gingivitis, periodontitis and other mucosal and gum diseases, that result from the bacterial activity in the oral cavity.

The present invention is therefore directed to a mouth-care product comprising an effective amount of at least one lantibiotic and at least one compound yielding fluoride ions, to reduce and/or prevent caries and/or gingivitis.

The present invention is also directed to a method to prevent the accumulation of dental plaque, thus reducing the formation of calculus and occurrence of caries, gingivitis and/or other periodontal diseases.

In a third aspect the present invention is directed to the use of lantibiotics in combination with at least one compound yielding fluoride ions, in mouth-care products to prevent the accumulation of dental plaque, thus reducing the formation of calculus and occurrence of caries, gingivitis and/or other periodontal diseases.

The lantibiotics which are useful in the mouth-care products according to the present invention are all presently known lantibiotics: nisin, subtilin, epidermin, gallidermin, pep 5, duramycin and duramycin B, cinnamycin, mersacidin, actagardin and ancovenin. These lantibiotics are described in the already cited article in Angew. Chem., Int. Ed. Engl., vol.30, no.9, 1053 (1991), but also other lantibiotics are expected to be suitable.

The preferred lantibiotics are nisin and subtilin; nisin is most preferred.

According to the invention an effective amount of one or more lantibiotics is present in a mouth-care product. An effective amount refers to an amount that performs an antibacterial and/or antimicrobial activity in the oral cavity during application of the mouth-care product according to the present invention. Such activity will in general result if a total amount of between 0.1 to 10000 ppm of one or more lantibiotics is added to the mouth-care product. Preferably the upper total amount of the lantibiotics does not exceed 1000 ppm. The total amount will preferably be more than 1 but less than 500 ppm and will most preferably be in the range of between 1 and 250 ppm for tooth pastes and dental gels, and the total amount will preferably be in the range of between 1 and 100 ppm in the case of tablets, toothpowder's, mouth washes and lozenges.

The presence of fluoride providing agents in the mouth-care products according to the present invention is important. The use of fluoride ions, or fluor-ion providing compounds as anti-caries agents is well known in the art. Suitable compounds may be slightly soluble in water or may be fully water-soluble. They are characterized by their ability to release fluoride ions in water and by freedom from undesired reaction with other compounds of the oral preparation. It was to be expected that the presence of fluoride would deactivate the lantibiotic. However, it has surprisingly been found that the combination of lantibiotic and fluoride enhances the effects against caries and gingivitis. Especially the use of alkalimetal fluorides, more in particular sodium fluoride, results in improved activity of the oral care products.

Among suitable compounds yielding fluoride ions are inorganic fluoride salts, such as soluble alkali metal and alkaline earth metal salts, for example sodium fluoride, potassium fluoride, barium fluoride, or calcium fluoride and ammonium fluoride, or a copper fluoride, or zinc fluoride, sodium fluorosilicate, ammonium fluorosilicate, sodium fluorozirconate, sodium mono-fluorophosphate, aluminium mono- and di-fluorophosphate, and fluorinated sodium calcium pyrophosphate. Alkali metal and tin fluorides, such as sodium and stannous fluorides, sodium monofluorophosphate, organic fluorides, such as aminfluoride and mixtures thereof, are preferred.

The amount of fluoride-providing compounds is dependent to some extent upon the type of compound, its solubility, and the type of oral preparation, but it must be a nontoxic, effective amount, generally about 0.005 to about 3.0% in the preparation, e.g. gel, tooth paste or tooth powder, an amount of such compound which releases up to about 5,000 ppm of F-ion by weight of the preparation is considered satisfactory. Any suitable minimum amount of such compound may be used, but it is preferable to employ sufficient compound to release about 300 to about 2,000 ppm, more preferably about 800 to about 1,500 ppm of fluoride ion. Typically, in the case of alkali metal fluorides and stannous fluoride, this component is present in an amount up to about 2% by weight, based on the weight of the preparation, and preferably in the range of about 0,05% to 1%. In the case of sodium monofluorophosphate, the compound may be present in an amount of about 0.1-3%. In dentrifrice preparations such as lozenges and chewing gum, the fluorine-providing compound is typically present in an amount sufficient to release up to about 500 ppm, preferably about 25 to about 300 ppm by weight of fluoride ion. Generally about 0.005 to about 1.0 wt% of such compound is present.

Lantibiotics in combination with fluoride are compatible to the usual mouth care products additives, such as abrasives, polishing agents, thickening agents, foaming agents, flavouring agents, sweeteners, preservatives and/or other basic ingredients.

The mouth-care product according to the present invention can have all suitable forms, like tooth paste or cream, dental gel, tooth powder, mouth-wash, chewing gum, dental or chewing tablet, lozenge or effervescent tablet or any other suitable form.

These products may contain the conventional components that are usually present in those products. The choice thereof depends on the actual type of product.

The mouth-care products according to the present invention can, if necessary, be based upon an orally acceptable vehicle.

In certain desirable forms of the present invention, the oral composition may be substantially solid or pasty in character, such as toothpowder, dental tablet, toothpaste or dental gel. Such solid or pasty oral preparations generally contains polishing material.

Examples of polishing materials are water-insoluble sodium metaphosphate, potassium metaphosphate, tricalcium phosphate, dehydrated calcium phosphate, anhydrous dicalcium phosphate, calcium pyrophosphate, magnesium orthophosphate, trimagnesium phosphate, aluminium oxide, aluminium oxide hydrate, calcium carbonate, aluminium silicate, zirconium silicate, silica, bentonite, and mixtures thereof. Other suitable polishing materials include the particulate thermosetting resins described in U.S. Patent No. 3 070 510, such as melamine-phenolic-, and urea-formaldehydes, and cross-linked polyepoxides and polyesters. Preferred polishing materials include silica having particle sizes of up to about 7 microns, a mean particle size of up to about 1.1 microns, and a surface area of up to about 50,000 cm2/gm, silica gel or colloidal silica, and complex amorphous alkali metal aluminosilicate.

When visually clear gels are desired, a polishing agent of colloidal silica, such as those sold under the trademark SYLOID as Syloid 72 and Syloid 74 or under the trademark SANTOCEL as Santocel 100 and alkali metal alumino-silicate complexes are particularly useful, since they have refractive indices close to the refractive indices of gelling agent-liquid (including water and/or humectant) systems commonly used in dentifrice's.

The polishing material is generally present in the solid or paste compositions in weight concentrations of about 10% to about 99%. Preferably, it is present in amounts ranging from about 10% to about 75% in toothpaste, and from about 70% to about 99% in toothpowder.

In a toothpaste, the liquid vehicle may comprise water and humectant typically in an amount ranging from about 10% to about 90% by weight of the preparation. Glycerine, propylene glycol, sorbitol, polypropylene glycol and/or polyethylene glycol exemplify suitable humectants/carriers. Also advantageous are liquid mixtures of water, glycerine and sorbitol. In clear gels where the refractive index is an important consideration, about 3-30 wt% of water, 0 to about 80 wt% of glycerine, and about 20-80 wt% of sorbitol is preferably employed.

Toothpaste's and gels typically contain a natural or synthetic thickener or gelling agent in proportions of about 0.1 to about 10, preferably about 0.5 to about 5 wt%. A suitable thickener is synthetic hectorite, a synthetic colloidal magnesium alkali metal silicate complex clay available for example as Laponite (e.g. CP, SP 2002, D) marketed by Laporte Industries Limited.

Other suitable thickeners include Irish moss, gum tragacanth, starch, polyvinylpyrrolidone, hydroxyethylpropyl cellulose, hydroxybutyl methyl cellulose, hydroxypropyl methyl cellulose, hydroxyethyl cellulose (e.g. available as Natrosol), sodium carboxymethyl cellulose, and colloidal silica such as finely ground Syloid.

Any suitable flavouring or sweetening material may also be employed. Examples of suitable flavouring constituents are flavouring oils, e.g. oil of spearmint, peppermint, wintergreen, sassafras, clove, sage, eucalyptus, marjoram, cinnamon, lemon, and orange, and methyl salicylate. Suitable sweetening agents include sucrose, lactose, maltose, sorbitol, xylitol, sodium cyclamate, perillartine, APM (aspartyl phenyl alanine methyl ester), saccharine and the like. Suitably, flavour and sweetening agents may together comprise from about 0.1% to 5% or more of the preparation.

The lantibiotics are compatible with various other materials, used as active components in oral care products, such as phosphates, such as trimetaphosphates and diammoniumphosphates, enzymes, anti-tartar agents such as pyrophosphates, zinc-compounds and phosphonates and/or other anti-plaque ingredients, such as triclosan, chlorhexidine, bromochlorophene and sanquinarine, agents for sensitive teeth, such as specific alkalimetal salts like strontium salts and/or potassium nitrate or - citrate, wound healing agents such as allantoin, chlorophyll, tocopherol and herbal extracts, activity enhancing agents, or boosters, as gantrez and some polymers, and specific ingredients as urea, xylitol, silicones, quaternary ammonium compounds and mixtures thereof. These components, as well as the amounts to be used, are known in the art. They are, where present, incorporated in the preparations in amounts which do not substantially adversely affect the properties and characteristics desired.

Examples of suitable additional anti-plaque agents are triclosan, chlorhexidine, bromochlorophene, sanguinarine, metal salts and xylitol. Anti-calculus agents can for example be chosen from pyrophosphate, zinc-compounds and diphosphonates.

Agents for sensitive dentures are usually specific akalimetal salts, like strontium salts and potassium nitrate, or -citrate. Wound healing agents can for example be allantoin, chlorophyl, tocopherol and herbal extracts. Finally the activity enhancing agents, or boosters, can be present to enhance the delivery and retention of the active components. Examples are Gantrez® and some polymers.

The use of the lantibiotics according to the present invention can advantageously be combined with the use of the enzyme systems that are disclosed in US patents Nos.4 150 113 and 4 871 532. According to these patents hydrogen peroxide producing enzym systems have advantageous effects on the reduction of oral bacterial activity. The most effective enzyme is the oxidoreductase enzyme glucose oxidase. This enzyme may be present in combination with other enzymes such as amyloglucosidase, dextranase and/or mutanase, optionally in the presence of zinc ion providing compounds and/or 8-hydroxyquinoline derivatives. Other enzymes like those disclosed in US patent 4 152 418 may also be present.

Such combination of enzymes and lantibiotics has a combined advantageous effect on the oral cavity condition, i.e., it will lead to a reduction of the bacterial activity to diminish the deposition of dental plaque and calculus formation.

Other enzymes like lactoperoxidase, lactoferrin and lysozyme may also be present and provide an oral care product having advantageous properties.

A preferred embodiment of the mouth-care product according to the present invention is a mouth-care product containing an effective amount of nisin as well as an effective amount of the said oxidoreductase enzyme system.

Another preferred embodiment of the mouth-care product according to the present invention is a tooth paste containing an effective amount of nisin, as well as an effective amount of fluoride.

The vehicle or carrier in a tablet or lozenge is a non-cariogenic solid water soluble polyhydric alcohol (polyol) such as mannitol, xylitol, sorbitol, maltitol, a hydrogenated starch hydrolysate, Lycasin, hydrogenated glucose, hydrogenated disaccharide's, and hydrogenated polysaccharides, in an amount of about 90-98% by weight of the total composition. Solid salts such as sodium carbonate, sodium chloride, potassium bicarbonate or potassium chloride may totally or partially replace the polyol carrier.

Tableting lubricants, in minor amounts of about 0.1 to 5% by weight, may be incorporated into the table or lozenge formulation to facilitate the preparation of both the tablets and lozenges. Suitable lubricants include vegetable oils such as coconut oil, magnesium stearate, aluminium stearate, talc, starch and carbowax.

The compositions of the present invention can be incorporated in lozenges, or in chewing gum or other products, e.g. by stirring into a warm gum base or coating the outer surface of a gum base, illustrative of which may be mentioned jelutone, rubber latex, vinylite resins, etc., desirable with conventional plasticizers or softeners, sugar or other sweeteners or carbohydrates such as glucose, sorbitol and the like.

Lozenge formulations may optionally contain about 2% gum as barrier agent to provide a shiny surface as opposed to a tablet which has a smooth finish. Suitable non-cariogenic gums include Kappa carrageenan, carboxymethyl cellulose, hydroxyethyl cellulose, Gantrez, and the like.

The lozenge or tablet may optionally be coated with a coating material such as waxes, shellac, carboxymethyl cellulose, polyethylene maleic anhydride copolymer or Kappa-carrageenan to further increase the time it takes the tablet or lozenge to dissolve in the mouth. The uncoated tablet or lozenge is slow dissolving, providing a sustained release rate of the lantibiotic agent. Accordingly, the solid dose tablet and lozenge compositions of the present invention affords a relatively longer time period of contact of the teeth in the oral cavity with the active ingredient.

The mouth-care product according to the present invention can be prepared by the conventional ways of preparing said mouth-care products. The lantibiotics can be added to the finished mouth-care product or can be added during the production process. The process parameters like temperatures and pressures may have the conventional values.

The pH of the dentrifice preparation of the invention is generally in the range of from about 4.5 to about 10 and typically from about 5.5 to 9. Surprisingly the combination of fluoride and lantibiotic results in an improved anti-bacterial activity over a broader range of pH.

The pH is preferably in the range of from about 5.5 to about 8.0. It is noteworthy that the compositions of the invention may be applied orally at the said pH ranges without substantially decalcifying or otherwise damaging dental enamel. The pH can be controlled with acid (e.g. citric acid or benzoic acid) or base (e.g. sodium hydroxide) or be buffered (e.g. with sodium citrate, benzoate, carbonate, or bicarbonate, disodium hydrogen phosphate, or sodium dihydrogen phosphate).

The invention will now be Illustrated by means of the following examples.

### Example 1

### In vitro activity of lantibiotics

The activity of the lantibiotics in saliva is shown by the following in vitro test.
Human saliva was brought into cups at 37°C, and mixed with 1 % glucose. The metabolic activity of the oral micro flora was assessed by the acid formation and ATP production. ATP stands for adenosine tri-phosphate, a compound containing a high-energy bond which is used by organisms to store the energy freed from catabolic processes. The ATP level is used as a measure of the bacterial activity in the saliva.

| Nisin concentration (ppm) in the saliva with 50 ppm F⁻ | Acid formation | ATP level |
|---|---|---|
| 0 | present | high |
| 10.0 | strongly diminished | low |
| 12.5 | absent | low |

This example illustrates that the addition of nisin with fluoride restrains the acid formation and reduces the ATP level in the saliva. The addition of nisin with fluoride thus lowers the metabolic activity of the oral bacteria, and hence the accumulation of plaque on the teeth will be reduced as well.

### Example 2

### Tooth paste

A tooth paste is prepared according to the method known in the art and consists of the following components:
(All amounts are percent by weight unless otherwise indicated)

| | |
|---|---|
| glycerol | 15.0 |
| aluminium oxide hydrate | 35.0 |
| ethoxylated fatty alcohol | 3.0 |
| carraghene | 1.75 |
| esters of para-hydroxy-benzoic acids (PHB) | 0.15 |
| aroma | 1.2 |
| sodium fluoride | 0.33 |
| nisin | 250 ppm |
| water to | 100 % |

### Example 3

### Mouth-wash

A mouth-wash is prepared according to the method known in the art and consists of the following components:
(All amounts are percent by weight unless otherwise indicated)

| | |
|---|---|
| glycerol | 15.0 |
| xylitol | 15.0 |
| aroma | 0.05 |
| Tween® 80 | 2.0 |
| propyl para hydroxy benzoate | 0.1 |
| sodium citrate | 0.2 |
| sodium fluoride | 0.1 |
| nisin | 100 ppm |
| water to | 100 % |

### Example 4

### Lozenge

A lozenge is prepared according to the method known in the art and consists of the following components:
(All amounts are in mg unless otherwise indicated)

| | |
|---|---|
| sorbitol | 1500 |
| encapsulated aroma | 10 |
| sodium stearate | 10 |
| phosphate buffer pH = 5.5 | 50 |
| poly vinyl pyrrolidone (PVP) | 20 |
| sodium fluoride | 1.0 |
| nisin | 100 ppm |

### Example 5

### Chewing tablet

A chewing tablet is prepared according to the method known in the art and consists of the following components:
(All amounts are in percent by weight unless otherwise indicated)

| | |
|---|---|
| gum base | 25 |
| sorbitol | 17.5 |
| mannitol | 5 |
| aroma | 0.5 |
| glycerol | 3.0 |
| xylitol | 10 |
| sodium fluoride | 0.01 |
| nisin | 50 ppm |
| sorbitol to | 100 % |

### Example 6

### Effervescent tablet

An effervescent tablet is prepared according to the method known in the art and consists of the following components: (All amounts are in mg unless otherwise indicated)

| | |
|---|---|
| sodium carbonate | 40 |
| citric acid | 20 |
| poly vinyl pyrrolidone (PVP) | 2 |
| sorbitol | 24 |
| encapsulated aroma | 2 |
| sodium fluoride | 10 |
| subtilin | 250 ppm |
| dissolve in: 15 ml water | |

### Example 7

### Tooth powder

A tooth powder is prepared according to the method known in the art and consists of the following components:
(All amounts are in percent by weight unless otherwise indicated)

| | |
|---|---|
| aroma | 2 |
| sodium saccharinate | 0.15 |
| detergent | 1 |
| sodium fluoride | 0.33 |
| nisin | 100 ppm |
| calcium phosphate to | 100 % |

### Example 8

### Tooth paste

A tooth paste is prepared according to the method known in the art and consists of the following components:
(All amounts are in percent by weight unless otherwise indicated)

| | |
|---|---|
| sorbitol | 22 |
| silica | 22 |
| ethoxylated fatty alcohol | 3.0 |
| carraghene | 1.5 |
| sodium benzoate | 0.1 |
| aroma | 0.6 |
| sodium saccharinate | 0.1 |
| amyloglucosidase | 18 U/g |
| glucose oxidase | 6 U/g |
| mutanase | 16 U/g |
| zinc gluconate | 8·10⁻⁴ |
| 5 chloro-8-hydroxyquinoline | 1·10⁻³ |
| sodium fluoride | 0.33 |
| subtilin | 150 ppm |
| water to | 100 % |

## Claims

1. Mouth-care product comprising an effective amount of at least one lantibiotic in combination with an effective amount of at least one fluoride-providing compound.

2. Mouth-care product according to claim 1, in which the said at least one lantibiotic is chosen from the group consisting of nisin, epidermin, subtilin, pep 5, duramycin, ancovenin and gallidermin.

3. Mouth-care product according to claim 2, in which the said at least one lantibiotic is nisin.

4. Mouth-care product according to claim 1-3, in which the fluoride compound comprises an alkalimetal fluoride, more in particular sodium fluoride.

5. Mouth-care product according to claim 1-4 in which the said at least one lantibiotic component is present in an amount of between 0.1 and 10,000 ppm.

6. Mouth-care product according to claim 5 in which the said at least one lantibiotic component is present in an amount of between 1 and 500 ppm, preferably in an amount of between 1 and 25 ppm.

7. Mouth-care product according to claim 1-6 in which abrasive agents, polishing agents, thickening agents, colouring agents, sweetening agents, flavouring agents, foaming agents and/or other active components are also present.

8. Mouth-care product according to claim 1-7 characterized in that it is in the form of a tooth paste or cream, dental gel, tooth powder, mouth-wash, chewing gum, dental or chewing tablet, lozenge or effervescent tablet.

9. Mouth-care product according to claim 1-8 in the form of a tooth paste in which the said at least one lantibiotic component is nisin.

## Patentansprüche

1. Mundpflegemittel, versehen mit einer wirksamen Menge von mindestens einem Lantibiotikum in Kombination mit einer wirksamen Menge mindestens einer Fluorid-liefernden Verbindung.

2. Mundpflegemittel nach Anspruch 1, wobei das mindestens eine Lantibiotikum aus der Gruppe gewählt ist, bestehend aus Nisin, Epidermin, Subtilin, Pep-5, Duramycin, Ancovenin und Gallidermin.

3. Mundpflegemittel nach Anspruch 2, wobei das mindestens eine Lantibiotikum Nisin ist.

4. Mundpflegemittel nach Ansprüchen 1 bis 3, wobei die Fluoridverbindung ein Alkalimetalifluorid, insbesondere Natriumfluorid, enthält.

5. Mundpflegemittel nach Ansprüchen 1 bis 4, wobei die mindestens eine Lantibiotikum-Komponente in einer Menge zwischen 0,1 und 10.000 ppm vorhanden ist.

6. Mundpflegemittel nach Anspruch 5, wobei die mindestens eine Lantibiotikum-Komponente in einer Menge zwischen 1 und 500 ppm, vorzugsweise in einer Menge zwischen 1 und 25 ppm, vorhanden ist.

7. Mundpflegemittel nach Ansprüchen 1 bis 6, wobei Schleifmittel, Poliermittel, Verdickungsmittel, Färbemittel, Süßstoffe, Geschmacksstoffe, Schaummittel und/oder andere wirksame Komponenten auch vorhanden sind.

8. Mundpflegemittel nach Ansprüchen 1 bis 7, dadurch gekennzeichnet, daß es in Form einer Zahnpaste oder -creme, eines Zahngels, eines Mundspülmittels, eines Kaugummis, einer Zahn- oder Kautablette, einer Pastille oder einer Brausetablette ist.

9. Mundpflegemittel nach Ansprüchen 1 bis 8 in Form einer Zahnpaste, in der die mindestens eine Lantibiotikum-Komponente Nisin ist.

## Revendications

1. Un produit d'hygiène buccale comprenant une quantité efficace d'au moins un lantibiotique en combinaison avec une quantité efficace d'au moins un composé apportant du fluor.

2. Un produit d'hygiène buccale suivant la revendication 1, dans lequel au moins un lantibiotique est sélectionné dans le groupe comprenant la nisine, l'épidermine, la subtiline, le pep 5, la duramycine, l'ancovénine et la gallidermine.

3. Un produit d'hygiène buccale suivant la revendication 2, dans lequel ledit au moins un lantibiotique est la nisine.

4. Un produit d'hygiène buccale suivant les revendications 1-3, dans lequel le composé fluoré comprend un fluorure de métal alcalin, plus particulièrement le fluorure de sodium.

5. Un produit d'hygiène buccale suivant les revendications 1-4, dans lequel ledit au moins un composant lantibiotique est présent en quantité comprise entre 0,1 et 10.000 ppm.

6. Un produit d'hygiène buccale suivant la revendication 5, dans lequel ledit au moins un composant lantibiotique est présent en quantité comprise entre 1 et 500 ppm, de préférence en quantité comprise entre 1 et 25 ppm.

7. Un produit d'hygiène buccale suivant les revendications 1-6, dans lequel des agents abrasifs, des agents de polissage, des épaississants, des agents colorants, des agents édulcorants, des agents aromatisants, des agents moussants et/ou d'autres composants actifs sont également présents.

8. Un produit d'hygiène buccale suivant les revendications 1-7, caractérisé en ce qu'il se présente sous la forme d'une pâte ou d'une crème dentifrice, de gel dentaire, de poudre dentifrice, de bain de bouche, de pâte à mâcher, de tablettes dentaires ou tablettes à mâcher, de pastilles ou de tablettes effervescentes.

9. Un produit d'hygiène buccale suivant les revendications 1-8, se présentant sous la forme d'une pâte dentifrice dans laquelle ledit au moins un composant lantibiotique est la nisine.
